# EUROPÄISCHE PATENTSCHRIFT

(11) **EP 2 837 369 B1**
(45) Veröffentlichungstag und Bekanntmachung des Hinweises auf die Patenterteilung: **13.06.2018**
(21) Anmeldenummer: 13004079.3
(22) Anmeldetag: 16.08.2013
(51) Int. Cl.: A61F 13/00, A61M 1/00

(54) **Abdichtelement für einen Wundverband zur Unterdrucktherapie**
Sealing element for a wound dressing for vacuum therapy
Elément d'étanchéité pour un pansement pour le traitement par dépressurisation

(43) Veröffentlichungstag der Anmeldung: 18.02.2015
(73) Patentinhaber: Paul Hartmann AG, 89522 Heidenheim (DE)
(72) Erfinder: Eckstein, Axel, 89522 Heidenheim (DE); Croizat, Pierre, 89542 Herbrechtingen (DE); Kirsten, Juana, 89129 Langenau (DE)

(56) Entgegenhaltungen:
- EP-A1- 0 142 262
- WO-A1-2011/135285
- WO-A1-2012/069794
- DE-A1- 4 433 450
- US-A1- 2013 066 284

## Beschreibung

Die Erfindung betrifft allgemein ein Abdichtelement zur Anwendung in einer Unterdrucktherapie einer Wunde. Insbesondere betrifft die Erfindung ein in Form von Streifen bereitgestelltes Abdichtelement zur Anwendung in einer Unterdrucktherapie einer Wunde umfassend einen Verbund aus einem Silikongel-Adhäsiv und einer Folie.

Die Behandlung von Wunden mittels Unterdruck ist zum Beispiel aus der internationalen Patentanmeldung WO1993/009727 bekannt und wird insbesondere bei chronischen Wunden der Haut angewandt. Aus dem Stand der Technik ist eine Vielzahl von Vorrichtungen und Verbandsmaterialien zum Einsatz in der Unterdrucktherapie bekannt. In allen Fällen ist jedoch ein luftdichter Verschluss der Wunde zu gewährleisten, damit ein Unterdruck im Wundbereich zur Förderung der Wundheilung aufrechterhalten werden kann. Die zurzeit eingesetzten Unterdrucktherapievorrichtungen umfassen für diesen Zweck häufig ein Abdeckmaterial in Form von einer wasserdampfdurchlässigen, luftundurchlässigen Folie mit einer einseitigen adhäsiven Beschichtung. Die Folie wird an der die Wunde umgebenden Haut befestigt und überdeckt dabei die Wunde beziehungsweise die in oder auf die Wunde platzierten Verbandslagen. An weitestgehend glatter und trockener Haut in der Wundumgebung ist die Folie prinzipiell luftdicht anbringbar. Häufig weist die Haut in der Wundumgebung jedoch Falten auf, in deren Zwischenräume die Folie nicht immer eindringen kann. So entstehen Hohlräume zwischen der Haut und der Folie. Gelangt über diese Hohlräume Luft in den Wundverband, bilden sich Undichtigkeiten aus. Diese Undichtigkeiten können die Unterdrucktherapievorrichtung darin beeinträchtigen, das Wundgewebe einem Unterdruck auszusetzen.

Um Undichtigkeiten in einem Unterdruckwundverband zu verschließen, sind bisher verschiedene Lösungen vorgeschlagen worden. So wird in der internationalen Patentanmeldung WO96/09022 die Verwendung eines leicht verformbaren, nicht fließenden Dichtungsmaterials zur luftdichten Versiegelung von Unterdruckwundverbänden beschrieben. Dabei wird das Dichtungsmaterial insbesondere zum Verschließen von Undichtigkeiten, welche infolge eines unterhalb der Abdeckfolie des Verbandes angeordneten Drainageschlauches entstehen, verwendet. Bei dem Dichtungsmaterial kann es sich um ein Silikon, Hydrokolloid oder Lyogel handeln, welches entweder in einer Tube oder in Form von mit einer Hüllfolie ummantelten Streifen bereitgestellt wird.

Produkte zur Abdichtung von Unterdruckwundverbänden sind kommerziell erhältlich. Häufig umfassen diese Produkte doppelseitig adhäsive Hydrogel-Streifen oder Abdichtmittel auf Basis aushärtender Silikone. Letztere werden beispielsweise mit Hilfe einer Spritze appliziert. Ein zwischen der Hautoberfläche in der Wundumgebung und dem Abdeckmaterial angeordnetes Abdichtmittel kann in manchen Fällen mit Wundexsudat in Berührung kommen. Hydrogele können dann aufquellen und müssen gegebenenfalls vorzeitig ausgetauscht werden. Die aushärtenden Silikone andererseits sind in der Regel nicht steril. Weiterhin kann es sich als schwierig erweisen, ein Silikon-Abdichtmittel mit einer Spritze so zu applizieren, dass das Silikon ausschließlich auf den gewünschten Hautbereich aufgetragen wird.

Der vorliegenden Erfindung liegt die Aufgabe zugrunde, die Unterdrucktherapie von Wunden weiter zu verbessern und die Nachteile im Stand der Technik zu überwinden. Insbesondere liegt der vorliegenden Erfindung die Aufgabe zugrunde, ein Mittel zum Abdichten eines Unterdruckverbandes zur Verfügung zu stellen, welches seine abdichtende Wirkung über einen langen Zeitraum, beispielsweise über zwei bis fünf Tage, beibehält und sich gleichzeitig durch eine besonders einfache und schnelle Handhabung auszeichnet. Weiterhin liegt der vorliegenden Erfindung die Aufgabe zugrunde, eine Vorrichtung für eine Unterdruckwundtherapie zur Verfügung zu stellen, um auch bei unebener Haut, beispielsweise Altershaut, in der Wundumgebung eine dauerhafte und gleichzeitig besonders einfach und schnell anwendbare luftdichte Abdeckung der Wunde zu erreichen. Die Erfindung löst die Aufgabe mit einem Abdichtelement mit den Merkmalen von Anspruch 1 oder mit einer Vorrichtung zum luftdichten Abdecken einer Wunde mit den Merkmalen von Anspruch 2.

Überraschend wurde gefunden, dass ein Abdichtelement mit den Merkmalen von Anspruch 1 besonders geeignet ist, Undichtigkeiten zwischen einer an eine Wunde angrenzenden Hautoberfläche und einem luftundurchlässigen Abdeckmaterial in einer Unterdrucktherapie zu verschließen. So geht die abdichtende Wirkung des erfindungsgemäßen Abdichtelementes auch bei intensiven Kontakt mit Wundexsudat nicht verloren. Mit dem erfindungsgemäßen Abdichtelement lassen sich die Undichtigkeiten zuverlässig und lang anhaltend, beispielsweise über zwei bis fünf Tage, verschließen. Weiterhin lassen sich die Undichtigkeiten mit dem erfindungsgemäßen Abdichtelement sehr einfach und schnell verschließen. Es besteht eine geringere Gefahr, dass das Abdichtelement an den Fingern des Anwenders haften bleibt oder auf sich selbst zum Kleben kommt. Vorteilhaft an dem Abdichtelement ist auch, dass es in steriler Form bereitgestellt und zugeschnitten werden kann. Indem das Abdichtelement zugeschnitten werden kann, lässt es sich präzise an die Größe der abzudichtenden Hautoberfläche anpassen.

Die Erfindung betrifft demnach gemäß einem ersten Aspekt ein Abdichtelement, welches in Form von Streifen bereitgestellt wird, zur Anwendung in einer Unterdrucktherapie einer Wunde, umfassend einen Verbund aus
a) einem Silikongel-Adhäsiv zum Verschließen von Undichtigkeiten zwischen einer an die Wunde angrenzenden Hautoberfläche und einem luftundurchlässigen Abdeckmaterial, wobei das Silikongel-Adhäsiv eine im Gebrauch der Hautoberfläche abgewandte erste Seite und eine im Gebrauch der Hautoberfläche zugewandte zweite Seite umfasst und wobei das Silikongel-Adhäsiv eine Schichtdicke von mindestens 1500 µm bis höchstens 3500 µm aufweist; und
b) einer ersten Folie, welche mit der ersten Seite des Silikongel-Adhäsives unlösbar verbunden ist und welche auf ihrer dem Silikongel-Adhäsiv abgewandten Seite nicht-adhäsiv ausgebildet ist;
wobei das Abdichtelement eine Adhäsionskraft von mindestens 8 N/25 mm und höchstens 20 N/25 mm, bevorzugt von mindestens 10 N/25 mm und höchstens 20 N/25 mm, mehr bevorzugt von mindestens 12 N/25 mm und höchstens 20 N/25 mm oder ganz besonders bevorzugt von mindestens 16 N/25 mm und höchstens 20 N/25 mm, gemessen analog DIN EN 1939 in Verbindung mit DIN ISO 6133:2004-05, aufweist. Hinsichtlich der Angabe "analog DIN EN 1939 in Verbindung mit DIN ISO 6133:2004-05" wird auf das weiter unten näher beschriebene Messverfahren zur Bestimmung der Adhäsionskraft (Klebkraft) Bezug genommen.

Die Adhäsionskraft des Abdichtelementes gemäß diesem ersten Aspekt der Erfindung muss also mindestens 8 N/25 mm und höchstens 20 N/25 mm, gemessen analog DIN EN 1939 in Verbindung mit DIN ISO 6133:2004-05, betragen. Bevorzugt weist das Abdichtelement eine Adhäsionskraft von mindestens 10 N/25 mm und höchstens 20 N/25 mm, mehr bevorzugt von mindestens 12 N/25 mm und höchstens 20 N/25 mm oder ganz besonders bevorzugt von mindestens 16 N/25 mm und höchstens 20 N/25 mm, gemessen analog DIN EN 1939 in Verbindung mit DIN ISO 6133:2004-05, auf.

Nach einer weiteren Ausführungsform gemäß dem ersten Aspekt der Erfindung beträgt die Adhäsionskraft des Abdichtelementes vorteilhafterweise mindestens 14 N/25 mm und höchstens 18 N/25 mm, vorzugsweise mindestens 14 N/25 mm und höchstens 16 N/25 mm, gemessen analog DIN EN 1939 in Verbindung mit DIN ISO 6133:2004-05.

Gemäß einem zweiten Aspekt betrifft die Erfindung eine Vorrichtung zum luftdichten Abdecken einer Wunde für eine Unterdrucktherapie umfassend
a) ein luftundurchlässiges Abdeckmaterial, welches derart an einer an die Wunde angrenzenden Hautoberfläche adhäsiv befestigbar ist, dass die Wunde überdeckbar ist;
b) ein Abdichtelement gemäß dem ersten Aspekt der Erfindung, welches im Gebrauch zwischen der an die Wunde angrenzenden Hautoberfläche und dem Abdeckmaterial vorhanden sein kann.

Unter Verwendung der erfindungsgemäßen Vorrichtung lässt sich auf einfache und zuverlässige Weise eine luftdichte Abdeckung einer Wunde für eine Unterdrucktherapie herstellen. Unter "luftdichter Abdeckung" soll hier nicht verstanden werden, dass keinerlei Gasaustausch zwischen dem Wundraum, also dem Raum zwischen der Oberfläche der Wunde und dem Abdeckmaterial, und seiner Umgebung stattfindet. Vielmehr ist mit "luftdichter Abdeckung" in diesem Zusammenhang gemeint, dass unter Berücksichtigung der eingesetzten Unterdruckquelle der für die Unterdrucktherapie von Wunden nötige Unterdruck aufrechterhalten werden kann. Es können deshalb auch Abdeckmaterialien eingesetzt werden, welche eine geringfügige Gas-Permeabilität aufweisen, so lange der für die Unterdrucktherapie notwendige Unterdruck aufrechterhalten werden kann. Der für die Therapie notwendige Unterdruck, worunter die Druckdifferenz zwischen dem Luftdruck innerhalb des Wundverbandes und dem Umgebungsluftdruck, angegeben als positiver Zahlenwert in mm Hg, zu verstehen ist, bewegt sich üblicherweise in einem Bereich von mindestens 80 mm Hg bis höchstens 150 mm Hg.

Das luftundurchlässige Abdeckmaterial kann aus einem festen Material, beispielsweise in Form einer Auflageplatte, bestehen oder als eine flexible Folie vorliegen. Kombinationen davon sind gleichfalls vorstellbar. Bevorzugt handelt es sich bei dem luftundurchlässigen Abdeckmaterial um eine Folie, welche ein wasserunlösliches Polymer oder ein Metall umfasst. Ein für die Folie des Abdeckmaterials geeignetes wasserunlösliches Polymer ist beispielsweise Polyurethan, Polyester, Polypropylen, Polyethylen, Polyamid oder Polyvinylchlorid, Polyorganosiloxan (Silikon), oder eine Mischung daraus.

Damit das luftundurchlässige Abdeckmaterial adhäsiv an der an die Wunde angrenzenden Hautoberfläche oder anders ausgedrückt an der die Wunde umgebenden Hautoberfläche befestigt werden kann, ist es auf einer Seite selbstklebend, vorzugsweise vollflächig selbstklebend, ausgestattet. Die Hautoberfläche, an welcher das Abdeckmaterial befestigt wird, ist typischerweise nicht verletzt.

Ein mögliches Abdeckmaterial für die erfindungsgemäße Vorrichtung ist Polyurethanfilm der Marke Hydrofilm® (Paul Hartmann AG, Deutschland) oder Visulin® (Paul Hartmann AG, Deutschland).

Das erfindungsgemäße Abdichtelement wird in Form von Streifen, also in Form von weitestgehend flachen, bevorzugt rechteckigen und weiterhin bevorzugt länglichen Abschnitten, bereitgestellt. In einer besonders bevorzugten Ausführungsform der Erfindung sind die Streifen des Abdichtelementes in der Aufsicht rechteckig mit den Abmessungen 5 cm x 10 cm bis 10 cm x 18 cm ausgebildet. Da das erfindungsgemäße Abdichtelement zuschneidbar ist, sind auch andere Formen möglich. Wenn im vorliegenden Dokument von "Bereitstellen" die Rede ist, wird Bezug auf den Zustand des Abdichtelementes vor dessen Anwendung, das heißt vor Anbringung an die Haut in der Wundumgebung, genommen.

Das erfindungsgemäße Abdichtelement umfasst einen Verbund aus einem Silikongel-Adhäsiv und einer ersten Folie, wobei das Silikongel-Adhäsiv Undichtigkeiten zwischen der an die Wunde angrenzenden Hautoberfläche und dem luftundurchlässigen Abdeckmaterial verschließen soll. Der Verbund des erfindungsgemäßen Abdichtelementes ist so gestaltet, dass das Silikongel-Adhäsiv nur auf seiner ersten Seite mit der ersten Folie unlösbar verbunden ist. Unter einer Undichtigkeit ist im vorliegenden Dokument allgemein ein Hohlraum zwischen einer an eine Wunde angrenzenden Hautoberfläche und einem adhäsiv ausgebildeten Abdeckmaterial zu verstehen, über den Luft in den Wundraum eindringt, so dass die Aufrechterhaltung des für die Unterdrucktherapie notwendigen Unterdrucks behindert wird.

Das Silikongel-Adhäsiv des erfindungsgemäßen Abdichtelementes ist weich und leicht verformbar. Dadurch kann das Silikongel zwischen abzudichtende Hautfalten eindringen. Die Schichtdicke des Silikongel-Adhäsives beträgt mindestens 1500 µm und höchstens 3500 µm. Eine derartige Schichtdicke des Silikongel-Adhäsives hat sich als besonders geeignet herausgestellt, um Undichtigkeiten zuverlässig zu verschließen. Da das Silikongel-Adhäsiv verformbar ist, kann sich dessen Schichtdicke nach der Applikation des Abdichtelementes auf einen faltigen Hautbereich verändern. Die Angaben im Hinblick auf die Schichtdicke des Silikongel-Adhäsives im vorliegenden Dokument beziehen sich daher stets auf das bereitgestellte Abdichtelement, also auf die Schichtdicke vor der Anwendung. Vorzugsweise umfasst das Silikongel-Adhäsiv ein Silikongel auf der Basis von Polydimethylsiloxan. Ein solches Silikongel lässt sich beispielsweise mit dem ZweiKomponenten-System Silbione® RT Gel 4512 A & B von der Firma Bluestar Silicones (Frankreich) herstellen.

Die Aufgabe der ersten Folie besteht im Wesentlichen darin die Anbringung des Abdichtelementes an die Haut des Patienten zu erleichtern. Entsprechend ist die erste Folie auf ihrer nicht mit dem Silikongel-Adhäsiv in Kontakt stehenden Seite nicht-adhäsiv beziehungsweise nicht-klebend ausgebildet. In einer bevorzugten Ausführungsform der Erfindung umfasst die erste Folie ein Polymer, insbesondere ein Polyurethan. Bevorzugt weist die erste Folie zudem eine Schichtdicke von mindestens 20 µm bis höchstens 60 µm auf.

Wie bereits dargestellt muss die Adhäsionskraft des erfindungsgemäßen Abdichtelementes mindestens 8 N/25 mm, gemessen analog DIN EN 1939 in Verbindung mit DIN ISO 6133:2004-05, betragen. Die Adhäsionskraft darf jedoch einen Wert von höchstens 20 N/25 mm nicht überschreiten, da sich das Abdichtelement nach der Wundtherapie ansonsten nur schwer von der Haut entfernen lässt. Bevorzugt weist das Abdichtelement eine Adhäsionskraft von mindestens 10 N/25 mm und höchstens 20 N/25 mm, mehr bevorzugt von mindestens 12 N/25 mm und höchstens 20 N/25 mm oder ganz besonders bevorzugt von mindestens 16 N/25 mm und höchstens 20 N/25 mm, gemessen analog DIN EN 1939 in Verbindung mit DIN ISO 6133:2004-05, auf. Die Adhäsionskraft kann vorteilhafterweise auch mindestens 14 N/25 mm und höchstens 18 N/25 mm, vorzugsweise mindestens 14 N/25 mm und höchstens 16 N/25 mm, gemessen analog DIN EN 1939 in Verbindung mit DIN ISO 6133:2004-05, betragen. Die vergleichsweise hohe Adhäsionskraft trägt dazu bei, dass Undichtigkeiten mit dem erfindungsgemäßen Abdichtelement zuverlässig und lang anhaltend verschlossen werden können.

Das Abdichtelement kann mit einer zweiten Folie, welche auf der zweiten Seite des Silikongel-Adhäsives lösbar aufgebracht ist, bereitgestellt werden. Diese zweite Folie kann ein Polymer, insbesondere Polyethylen, umfassen. Die Schichtdicke der zweiten Folie beträgt vorzugsweise mindestens 100 µm und höchstens 120 µm. Bei der zweiten Folie handelt es sich um eine Abziehfolie, welche das Silkongel-Adhäsiv während der Lagerung vor Verschmutzung und unerwünschter Haftung bewahrt. Damit sich die zweite Folie leicht von dem Silikongel-Adhäsiv ablösen lässt, kann sie zum Beispiel eine größere Fläche als das Adhäsiv aufweisen, geteilt sein und/oder Griffleisten umfassen.

Es wäre möglich, auf die nicht-adhäsiv ausgebildete Seite der ersten Folie eine weitere Folie (Trägerfolie) zur Stabilisierung des Abdichtelementes aufzubringen. Es hat sich jedoch als vorteilhaft herausgestellt, das Abdichtelement ohne eine derartige weitere Folie bereitzustellen. Eine Trägerfolie zur Stabilisierung ist für das erfindungsgemäße Abdichtelement nämlich nicht erforderlich, da die erste Folie durch das eine vergleichsweise hohe Schichtdicke aufweisende Silikongel-Adhäsiv bereits ausreichend stabilisiert wird. Indem auf eine weitere Folie zur Stabilisierung verzichtet wird, lässt sich das Abdichtelement schneller und exakter an der Haut in der Wundumgebung anbringen.

In einer besonders bevorzugten Ausführungsform der Erfindung wird das Abdichtelement in einem Beutel verpackt und steril bereitgestellt. Als Verpackungsmaterial eignet sich beispielsweise eine Aluminiumverbundfolie. Die Sterilisation des in einem Beutel verpackten Abdichtelementes wird vorzugsweise mit Strahlung, insbesondere Gammastrahlung, durchgeführt.

Die Vorrichtung gemäß dem zweiten Aspekt der Erfindung kann weiterhin die folgenden Komponenten, welche typischerweise für die Durchführung einer Unterdrucktherapie benötigt werden, umfassen:
a) eine Wundauflage, welche auf die Oberfläche der Wunde aufbringbar ist, so dass die Wundauflage im Gebrauch zwischen der Oberfläche der Wunde und dem Abdeckmaterial angeordnet ist,
b) eine Unterdruckquelle, und
c) optional Mittel zur funktionellen Verbindung des Raumes, welcher zwischen der Oberfläche der Wunde und dem Abdeckmaterial gebildet wird, mit der Unterdruckquelle, so dass ein Unterdruck in dem Raum zwischen der Oberfläche der Wunde und dem Abdeckmaterial herstellbar ist, so dass von der Oberfläche der Wunde abgesonderte Flüssigkeiten aus dem Raum zwischen der Oberfläche der Wunde und dem Abdeckmaterial entfernbar beziehungsweise absaugbar sind.

Für den Raum, welcher zwischen der Oberfläche der Wunde und dem Abdeckmaterial gebildet wird, findet im vorliegenden Dokument auch der Begriff Wundraum Verwendung.

Geeignete Wundauflagen (Komponente a), Unterdruckquellen (Komponente b) und Mittel zur funktionellen Verbindung des Wundraumes mit der Unterdruckquelle (Komponente c) sind dem Fachmann bekannt. Exemplarisch wird dabei auf die internationalen Patentanmeldungen WO2011/003521, WO2011/018133, WO2011/076340 und WO2012/022485 der Anmelderin verwiesen. Die Wundauflage a) kann beispielsweise einen SAP-haltigen Saugkörper, einen offenzelligen oder halboffenzelligen Schaumstoff, insbesondere einen Polyurethanschaumstoff, ein Abstandsgewirke, eine Textilschicht, ein strukturiertes Gel, oder eine durchlässige Non-woven-Schicht umfassen. Die Unterdruckquelle b) der vorgenannten Vorrichtung umfasst vorzugsweise eine elektrisch betriebene Unterdruckpumpe und einen Auffangbehälter für die abgesaugten Wundflüssigkeiten, welche beide außerhalb des luftundurchlässigen Abdeckmaterials positioniert sind. Als Mittel zur funktionellen Verbindung des Wundraumes mit der Unterdruckquelle (Komponente c) kann eine Verbindungsleitung, beispielsweise ein Silikon-Drainageschlauch, und zusätzlich gegebenenfalls ein Unterdruckanschluss-Stück (Port) verwendet werden. Es ist gleichfalls denkbar, dass die Unterdruckquelle b) in Form einer Mikropumpe unterhalb oder direkt auf dem luftundurchlässigen Abdeckmaterial positioniert wird. In einem solchen Fall ist es unter Umständen möglich, auf die Verbindungsmittel c) zu verzichten.

Die anspruchsgemäße Adhäsionskraft (Klebkraft) des Abdichtelementes beziehungsweise des Verbundes aus dem Silikongel-Adhäsiv und der ersten Folie wird im Rahmen der vorliegenden Erfindung analog dem Verfahren 1 "Klebebänder - Messung der Klebkraft auf nicht rostendem Stahl unter einem Winkel von 180°" der deutschen Norm DIN EN 1939 (Ref. Nr. EN 1939:2003 D) in Verbindung mit der deutschen Norm DIN ISO 6133:2004-05 bestimmt. Hierbei werden sämtliche in den vorgenannten Normen angegebenen Parameter eingehalten, insofern im Folgenden nicht anders spezifiziert.

Abweichend von der Norm wird das zu prüfende Abdichtelement in Form von Streifen und nicht auf einer Rolle zur Anwendung bereitgestellt. Entsprechend sind die Angaben in der DIN EN 1939, welche festlegen wie der Probestreifen von einer Probenrolle zu entnehmen ist, nicht zu berücksichtigen. Das Abdichtelement wird auf eine Breite von 25 mm ± 0,25 mm und eine Länge von 300 mm ± 1 mm zugeschnitten, wobei gegebenenfalls vorhandenes Verpackungsmaterial zuvor entfernt wird. Das zugeschnittene Abdichtelement wird im Folgenden auch als Probestreifen bezeichnet. Die Konditionierung und die Prüfung des Probestreifens erfolgt wie in der Norm DIN EN 1939 (Punkt 5.4.1) dargestellt unter Normbedingungen, das heißt bei 23 °C ± 1 °C und 50 % ± 5 % relativer Luftfeuchte. Die Konditionierung erfolgt über einen Zeitraum von 24 Stunden. Falls das zu prüfende Abdichtelement keine zweite Folie zum Schutz des Silikongel-Adhäsives umfasst, wird das Abdichtelement während der Konditionierung so gelagert, dass die erste Folie des Abdichtelementes auf der Unterlage liegt und das Silikongel-Adhäsiv somit nach oben zeigt. Die weitere Verwendung erfolgt innerhalb von 5 Minuten nach Abschluss der Konditionierung. Falls das zu prüfende Abdichtelement eine zweite Folie zum Schutz des Silikongel-Adhäsives umfasst, wird diese zweite Folie während der Konditionierung auf dem Abdichtelement belassen. In diesem Falle wird die zweite Folie unmittelbar nach der Konditionierung entfernt. Die weitere Verwendung erfolgt dann gleichfalls innerhalb von 5 Minuten.

Zur weiteren Verwendung wird der Probestreifen wie in der DIN EN 1939 angegeben auf die normgemäß vorbereitete Prüfplatte aufgebracht und an der Zugprüfmaschine befestigt (Punkt 5.5.2.1, 5.5.3.1 und 5.5.3.2). Abweichend von der in der DIN EN 1939 (Punkt 5.5.3.1) gemachten Angabe wird nach dem Anrollen des Probestreifens an die Stahlplatte jedoch 10 Minuten gewartet, bevor die Messung an der Zugprüfmaschine begonnen wird. Außerdem werden abweichend von der Angabe in der DIN EN 1939 (Punkt 5.5.3.2, 2. Absatz) alle ermittelten Messwerte in einem Diagramm, welches auf der x-Achse die Zeit oder den Weg und auf der y-Achse die Kraft abbildet, dargestellt und die Adhäsionskraft in N/25 mm gemäß DIN ISO 6133:2004-05 bestimmt.

### Figuren

Nachstehend werden das erfindungsgemäße Abdichtelement und die erfindungsgemäße Vorrichtung zum luftdichten Abdecken einer Wunde für eine Unterdrucktherapie anhand von Zeichnungen näher erläutert. Die Erfindung soll nicht auf die in den Zeichnungen oder in der Beschreibung der Zeichnungen dargestellten Ausgestaltungen reduziert verstanden werden. Vielmehr umfasst die Erfindung auch Kombinationen der Einzelmerkmale der alternativen Formen.
**Figur 1** zeigt eine bevorzugte Ausführungsform des erfindungsgemäßen Abdichtelementes (schematische Seitenansicht).
**Figur 2** zeigt eine bevorzugte Ausführungsform des erfindungsgemäßen Abdichtelementes (schematische Aufsicht).
**Figur 3** zeigt eine bevorzugte Ausführungsform der erfindungsgemäßen Vorrichtung (schematische Seitenansicht).

### Figurenlegende

- 1: nicht-adhäsive Seite der ersten Folie
- 2: erste Folie
- 3: erste Seite des Silikongel-Adhäsives
- 4: Silikongel-Adhäsiv
- 5: zweite Seite des Silikongel-Adhäsives
- 6: zweite Folie
- 7: an die Wunde angrenzende Hautoberfläche
- 8: faltiger Bereich der an die Wunde angrenzenden Hautoberfläche
- 9: ebener Bereich der an die Wunde angrenzenden Hautoberfläche
- 10: Abdeckmaterial
- 11: Wundraum
- 12: Oberfläche der Wunde
- 13: Wundauflage
- 14: Unterdruckanschluss-Stück (Port)
- 15: Drainageschlauch
- 16: Unterdruckquelle

### Beschreibung der Figuren

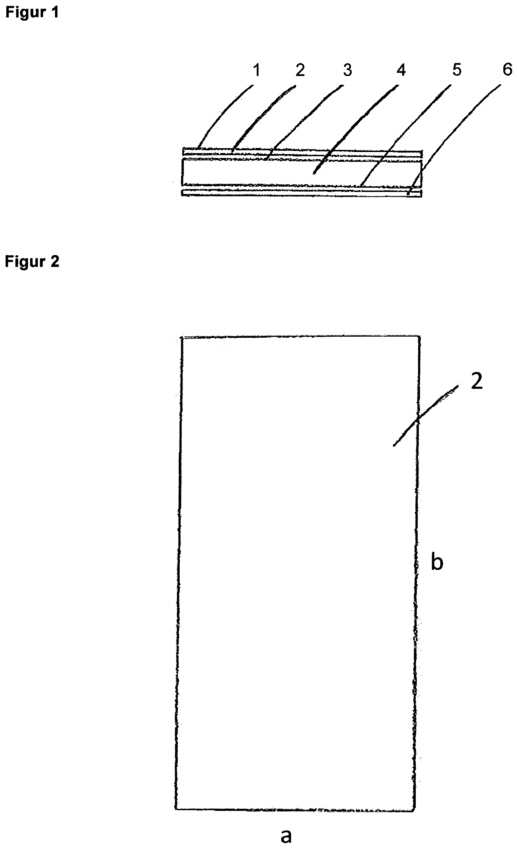

Figur 1 zeigt eine bevorzugte Ausführungsform des erfindungsgemäßen Abdichtelementes in einer schematischen Seitenansicht. Dabei wird das Abdichtelement vor dessen Anwendung dargestellt. Das Abdichtelement hat die Form eines Streifens und umfasst einen Verbund aus einem weichen und leicht verformbaren Silikongel-Adhäsiv (4) auf der Basis von Polydimethylsiloxan und einer ersten Folie (2) aus Polyurethan. Das Silikongel-Adhäsiv (4) umfasst eine erste Seite (3) und eine zweite Seite (5) und weist eine Schichtdicke von mindestens 1500 µm bis höchstens 3500 µm auf. Die erste Folie (2) ist mit der ersten Seite (3) des Silikongel-Adhäsives unlösbar verbunden und auf ihrer dem Silikongel-Adhäsiv abgewandten Seite (1) nicht-adhäsiv ausgebildet. Der ersten Folie (2) liegt keine Folie zur Stabilisierung auf. Allerdings umfasst das Abdichtelement eine zweite Folie (6) aus Polyethylen, welche auf der zweiten Seite (5) des Silikongel-Adhäsives zu dessen Schutz lösbar aufgebracht ist. Diese zweite Folie (6) wird bei der Anwendung des Abdichtelementes entfernt. Die Schichtdicke der ersten Folie (2) beträgt mindestens 20 µm bis höchstens 60 µm und die Schichtdicke der zweiten Folie (6) beträgt mindestens 100 µm bis höchstens 120 µm. Insgesamt ist das Abdichtelement somit weitestgehend flach. Das Abdichtelement beziehungsweise der Verbund aus dem Silikongel-Adhäsiv (4) und der ersten Folie (2) zeichnet sich weiterhin durch eine Adhäsionskraft von mindestens 8 N/25 mm und höchstens 20 N/25 mm, bevorzugt von mindestens 10 N/25 mm und höchstens 20 N/25 mm, mehr bevorzugt von mindestens 12 N/25 mm und höchstens 20 N/25 mm oder ganz besonders bevorzugt von mindestens 16 N/25 mm und höchstens 20 N/25 mm, gemessen analog DIN EN 1939 in Verbindung mit DIN ISO 6133:2004-05, aus.

In Figur 2 wird das Abdichtelement aus Figur 1 in der Aufsicht mit Blick auf die erste Folie (2) dargestellt. Man erkennt, dass das Abdichtelement rechteckig geformt ist. Diese rechteckige Form wird für das erfindungsgemäße Abdichtelement bevorzugt, wobei die Abmessungen des Abdichtelementes dann beispielsweise 5 cm x 10 cm bis 10 cm x 18 cm betragen können. Der erste Wert bezieht sich dabei auf die Seite a ("Breite") und der zweite Wert auf die Seite b ("Länge") des rechteckigen Abdichtelementes. In dem in Figur 2 abgebildeten Abdichtelement sind die Seiten a und b unterschiedlich lang ausgebildet, so dass ein länglicher Abschnitt für das Abdichtelement erhalten wird.

Die in den Figuren 1 und 2 gezeigte bevorzugte Ausführungsform des erfindungsgemäßen Abdichtelementes wird darüber hinaus in einem Beutel, zum Beispiel aus einer Aluminiumverbundfolie, verpackt und steril bereitgestellt. Der Beutel ist in den Figuren 1 und 2 nicht dargestellt.

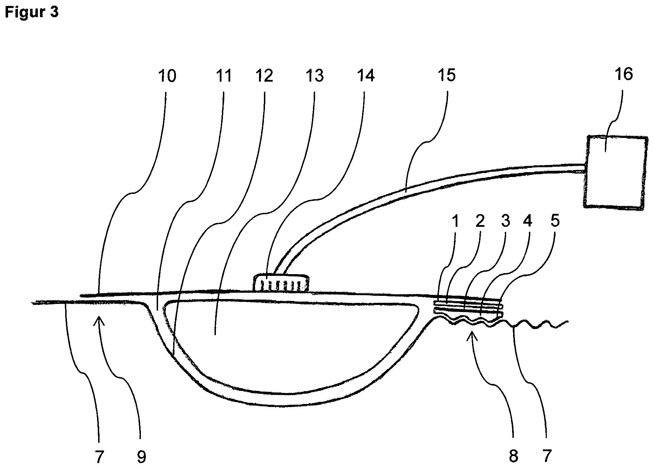

In Figur 3 ist eine bevorzugte Ausführungsform der erfindungsgemäßen Vorrichtung zum luftdichten Abdecken einer Wunde für eine Unterdrucktherapie zusammen mit weiteren, in der Unterdrucktherapie üblicherweise eingesetzten Komponenten abgebildet. Diese Figur veranschaulicht in stark schematisierter Form, wie ein Unterdruckverband mit dem erfindungsgemäßen Abdichtelement beschaffen sein kann. Das Abdichtelement wird in dem gezeigten Ausführungsbeispiel verwendet, damit das Abdeckmaterial ohne Ausbildung von Undichtigkeiten an einen faltigen Bereich (8) der an die Wunde angrenzenden Hautoberfläche befestigt werden kann. Der ebene Bereich (9) der an die Wunde angrenzenden Hautoberfläche wird bei der in Figur 3 dargestellten Ausführungsform nicht mit dem Abdichtelement in Kontakt gebracht, da sich das Abdeckmaterial alleine ohne Ausbildung von Undichtigkeiten an der ebenen Haut befestigen lässt.

Die Vorrichtung aus Figur 3 umfasst ein luftundurchlässiges Abdeckmaterial (10), welches derart an einer an die Wunde angrenzenden Hautoberfläche (7, 8, 9) adhäsiv befestigt wird, dass die Wunde überdeckt wird. Bei dem luftundurchlässigen Abdeckmaterial (10) handelt es sich um eine einseitig vollflächig selbstklebende Polyurethanfolie. Die Vorrichtung umfasst weiterhin ein zwischen der faltigen Hautoberfläche (8) und dem Abdeckmaterial (10) eingebrachtes Abdichtelement, welches in Form von Streifen, beispielsweise wie in den Figuren 1 und 2 dargestellt, bereitgestellt wird. Das zwischen der faltigen Hautoberfläche (8) und dem Abdeckmaterial (10) eingebrachte Abdichtelement umfasst einen Verbund aus einem weichen und leicht verformbaren Silikongel-Adhäsiv (4) auf der Basis von Polydimethylsiloxan und einer ersten Folie (2) aus Polyurethan. Das Silikongel-Adhäsiv (4) umfasst eine erste, im Gebrauch der Hautoberfläche abgewandte Seite (3) und eine zweite, im Gebrauch der Hautoberfläche zugewandte Seite (5) und weist eine Schichtdicke von mindestens 1500 µm bis höchstens 3500 µm auf. Die erste Folie (2) ist mit der ersten Seite (3) des Silikongel-Adhäsives unlösbar verbunden und auf ihrer dem Silikongel-Adhäsiv abgewandten Seite (1) nicht-adhäsiv ausgebildet. Die Schichtdicke der ersten Folie (2) beträgt mindestens 20 µm bis höchstens 60 µm. Das Abdichtelement beziehungsweise der Verbund aus dem Silikongel-Adhäsiv (4) und der ersten Folie (2) zeichnet sich weiterhin durch eine Adhäsionskraft von mindestens 8 N/25 mm und höchstens 20 N/25 mm, bevorzugt von mindestens 10 N/25 mm und höchstens 20 N/25 mm, mehr bevorzugt von mindestens 12 N/25 mm und höchstens 20 N/25 mm oder ganz besonders bevorzugt von mindestens 16 N/25 mm und höchstens 20 N/25 mm, gemessen analog DIN EN 1939 in Verbindung mit DIN ISO 6133:2004-05, aus.

Die Vorrichtung aus Figur 3 umfasst zudem eine Wundauflage (13), welche zwischen der Oberfläche der Wunde (12) und dem Abdeckmaterial (10) eingebracht wird. Diese Wundauflage (13) kann ein offenzelliger, retikulierter Polyurethanschaumstoff sein. Aufgabe der Wundauflage (13) ist unter anderem den Wundraum (11) auszufüllen, den Unterdruck zu verteilen sowie die Ableitung des Wundexsudates zu unterstützen. In vielen Fällen wird nicht nur eine Wundauflage (13), sondern eine Kombination verschiedener Wundauflagen verwendet. Zum Beispiel werden häufig atraumatische Wundkontaktschichten mit Schaumstoffen kombiniert, um ein Verwachsen des Schaumstoffes mit der Wundoberfläche (12) zu verhindern. Ferner umfasst die Vorrichtung eine außerhalb des Abdeckmaterials (10) und somit außerhalb des Wundraumes (11) positionierte Unterdruckquelle (16). Bei der Unterdruckquelle (16) handelt es sich vorzugsweise um eine elektrische Unterdruckpumpe mit einem Auffangbehälter für die abgesaugten Wundflüssigkeiten. Schließlich umfasst die Vorrichtung noch Mittel (14, 15) zur funktionellen Verbindung des Wundraumes (11) mit der Unterdruckquelle (16), nämlich ein Unterdruckanschluss-Stück (14) in Verbindung mit einem Drainageschlauch (15). Die Verbindungsmittel (14, 15) sind erforderlich, damit ein Unterdruck in dem Wundraum (11) hergestellt werden kann und von der Wunde abgesonderte Flüssigkeiten aus dem Wundraum (11) entfernt beziehungsweise absaugt werden können. Das Unterdruckanschluss-Stück (14) wird über einer in dem Abdeckmaterial (10) befindlichen Öffnung (nicht in der Figur 3 dargestellt) luftdicht angebracht und steht dadurch mit dem Wundraum (11) in fluider Kommunikation.

### Anwendungsbeispiel

Wundtyp: Druckgeschwür; die an die Wunde angrenzende Hautoberfläche weist einen faltigen Bereich auf
Art des Abdichtmittels: Zur Abdichtung des Unterdruckverbandes wird die bevorzugte Ausführungsform des erfindungsgemäßen Abdichtelementes nach Figur 1 und 2 in der Abmessung 5 cm x 10 cm, mit einer Schichtdicke des Silikongel-Adhäsives von 2000 µm und einer Schichtdicke der ersten Folie von 40 µm verwendet. Die Adhäsionskraft des verwendeten Abdichtelementes beträgt 10 N/25 mm (gemessen analog DIN EN 1939 in Verbindung mit DIN ISO 6133:2004-05).
Art des Abdeckmaterials: einseitig vollflächig selbstklebende, luftundurchlässige Polyurethanfolie (Hydrofilm®, Paul Hartmann AG, Deutschland)

Zunächst wird die Wunde von Nekrosen und Kontaminationen befreit (Debridement) und gegebenenfalls gespült. In die Wunde wird dann ein offenzelliger, retikulierter Polyurethanschaumstoff (VivanoMed Foam, Paul Hartmann AG, Deutschland) passender Größe eingebracht. Die Wunde wird nun luftdicht abgedeckt, wofür zunächst das Abdichtelement auf den faltigen Bereich der Hautoberfläche geklebt wird. Dazu wird der als Verpackung für das Abdichtelement dienende Beutel geöffnet und das Abdichtelement falls erforderlich zugeschnitten. Danach wird die das Silikongel-Adhäsiv schützende Abziehfolie (zweite Folie) vom Abdichtelement entfernt und das Abdichtelement auf die abzudichtende, faltige Hautoberfläche geklebt. Je nach Art und Größe der unebenen und abzudichtenden Hautoberfläche kann ein Abschnitt des Abdichtelementes genügen oder es müssen mehrere Abschnitte nebeneinander oder auch übereinander auf die Hautoberfläche angebracht werden. Da das Abdichtelement stets auf mindestens einer Seite nicht-adhäsiv ausgebildet ist, lässt es sich gut handhaben und in einfacher Weise an den faltigen Bereich der Hautoberfläche anbringen. Im Anschluss wird die Polyurethanfolie an der an die Wunde angrenzenden Hautoberfläche beziehungsweise an dem Abdichtelement befestigt. Ein ca. 2 cm großes Loch wird in die Polyurethanfolie geschnitten und darüber ein Unterdruckanschluss-Stück (VivanoTec Port, Paul Hartmann AG, Deutschland) angebracht. Das Loch wird üblicherweise über dem zentralen Bereich der Wunde beziehungsweise des Polyurethanschaumes in die Folie geschnitten. Schließlich wird das Unterdruckanschluss-Stück über einen Drainageschlauch mit einer Unterdruckquelle (VivanoTec Unterdrucktherapieeinheit mit Sekretbehälter, Paul Hartmann AG, Deutschland) verbunden und die Therapie eingeleitet. Nach zwei Tagen wird der Verband gewechselt und dabei auch das Silikonabdichtelement vorsichtig von der Haut entfernt ohne diese dabei zu beschädigen. Danach kann gegebenenfalls ein neuer Unterdruckverband angelegt und die Unterdrucktherapie fortgesetzt oder ein anderes geeignetes Behandlungsverfahren bis zum Abheilen der Wunde angewandt werden.

Der Unterdruckverband kann auch in anderer Art und Weise mit dem Abdichtelement versehen werden. Zum Beispiel kann der Verband wie in der zuvor beschriebenen Weise, jedoch zunächst ohne das Abdichtelement angebracht werden. Bei Start der Unterdrucktherapie wird beobachtet, an welchen Stellen sich zwischen Hautoberfläche und Abdeckmaterial Undichtigkeiten ausbilden. Die Unterdruckquelle wird deaktiviert und das Abdeckmaterial von der Hautoberfläche soweit gelöst, dass die abzudichtenden Bereiche zugänglich sind. Schließlich wird das Abdichtelement auf die abzudichtenden Bereiche geklebt, das Abdeckmaterial wieder befestigt und die Unterdruckquelle aktiviert. Diese Vorgehensweise bietet sich an, wenn der Anwender im Zweifel darüber ist, ob oder wo sich Undichtigkeiten zwischen der Hautoberfläche und dem Abdeckmaterial ausbilden.

## Patentansprüche

1. Abdichtelement, welches in Form von Streifen bereitgestellt wird, zur Anwendung in einer Unterdrucktherapie einer Wunde, umfassend einen Verbund aus
a) einem Silikongel-Adhäsiv (4) zum Verschließen von Undichtigkeiten zwischen einer an die Wunde angrenzenden Hautoberfläche (7) und einem luftundurchlässigen Abdeckmaterial (10), wobei das Silikongel-Adhäsiv (4) eine im Gebrauch der Hautoberfläche abgewandte erste Seite (3) und eine im Gebrauch der Hautoberfläche zugewandte zweite Seite (5) umfasst und wobei das Silikongel-Adhäsiv (4) eine Schichtdicke von mindestens 1500 µm bis höchstens 3500 µm aufweist; und
b) einer ersten Folie (2), welche mit der ersten Seite (3) des Silikongel-Adhäsives (4) unlösbar verbunden ist und welche auf ihrer dem Silikongel-Adhäsiv abgewandten Seite (1) nicht-adhäsiv ausgebildet ist;
wobei das Abdichtelement eine Adhäsionskraft von mindestens 8 N/25 mm und höchstens 20 N/25 mm, bevorzugt von mindestens 10 N/25 mm und höchstens 20 N/25 mm, mehr bevorzugt von mindestens 12 N/25 mm und höchstens 20 N/25 mm oder ganz besonders bevorzugt von mindestens 16 N/25 mm und höchstens 20 N/25 mm, gemessen analog DIN EN 1939 in Verbindung mit DIN ISO 6133:2004-05, aufweist.

2. Vorrichtung zum luftdichten Abdecken einer Wunde für eine Unterdrucktherapie umfassend
a) ein luftundurchlässiges Abdeckmaterial (10), welches derart an einer an die Wunde angrenzenden Hautoberfläche (7) adhäsiv befestigbar ist, dass die Wunde überdeckbar ist;
b) ein Abdichtelement nach Anspruch 1, welches im Gebrauch zwischen der an die Wunde angrenzenden Hautoberfläche (7) und dem Abdeckmaterial (10) vorhanden sein kann.

3. Abdichtelement oder Vorrichtung nach Anspruch 1 oder 2, wobei es sich bei dem luftundurchlässigen Abdeckmaterial (10) um eine Folie umfassend ein wasserunlösliches Polymer, insbesondere Polyurethan, oder ein Metall handelt.

4. Abdichtelement oder Vorrichtung nach einem der vorangehenden Ansprüche, wobei die Streifen in der Aufsicht rechteckig mit den Abmessungen 5 cm x 10 cm bis 10 cm x 18 cm ausgebildet sind.

5. Abdichtelement oder Vorrichtung nach einem der vorangehenden Ansprüche, wobei das Silikongel-Adhäsiv (4) ein Silikongel auf der Basis von Polydimethylsiloxan umfasst.

6. Abdichtelement oder Vorrichtung nach einem der vorangehenden Ansprüche, wobei die erste Folie (2) ein Polymer, insbesondere Polyurethan, umfasst.

7. Abdichtelement oder Vorrichtung nach einem der vorangehenden Ansprüche, wobei die erste Folie (2) eine Schichtdicke von mindestens 20 µm bis höchstens 60 µm aufweist.

8. Abdichtelement oder Vorrichtung nach einem der vorangehenden Ansprüche, wobei das Abdichtelement mit einer zweiten Folie (6), welche auf der zweiten Seite (5) des Silikongel-Adhäsives (4) lösbar aufgebracht ist, bereitgestellt wird.

9. Abdichtelement oder Vorrichtung nach Anspruch 8, wobei die zweite Folie (6) ein Polymer, insbesondere Polyethylen, umfasst.

10. Abdichtelement oder Vorrichtung nach Anspruch 8 oder 9, wobei die zweite Folie (6) eine Schichtdicke von mindestens 100 µm bis höchstens 120 µm aufweist.

11. Abdichtelement oder Vorrichtung nach einem der vorangehenden Ansprüche, wobei das Abdichtelement derart bereitgestellt wird, dass der ersten Folie (2) keine weitere Folie zur Stabilisierung aufliegt.

12. Abdichtelement oder Vorrichtung nach einem der vorangehenden Ansprüche, wobei das Abdichtelement in einem Beutel verpackt und steril bereitgestellt wird.

13. Vorrichtung nach einem der Ansprüche 2 bis 12, wobei die Vorrichtung weiterhin
a) eine Wundauflage (13), welche auf die Oberfläche der Wunde (12) aufbringbar ist, so dass die Wundauflage im Gebrauch zwischen der Oberfläche der Wunde (12) und dem Abdeckmaterial (10) angeordnet ist,
b) eine Unterdruckquelle (16), und
c) optional Mittel (14, 15) zur funktionellen Verbindung des Raumes (11), welcher zwischen der Oberfläche der Wunde (12) und dem Abdeckmaterial (10) gebildet wird, mit der Unterdruckquelle (16), so dass ein Unterdruck in dem Raum (11) zwischen der Oberfläche der Wunde (12) und dem Abdeckmaterial (10) herstellbar ist, so dass von der Oberfläche der Wunde (12) abgesonderte Flüssigkeiten aus dem Raum (11) zwischen der Oberfläche der Wunde (12) und dem Abdeckmaterial (10) entfernbar sind, umfasst.

## Claims

1. Sealing element, provided in the form of strips, for application in a negative-pressure therapy of a wound, comprising a composite of
a) a silicone gel adhesive (4) for closing off gaps between a skin surface (7) bordering the wound and an air-impermeable covering material (10), where the silicone gel adhesive (4) comprises a first side (3), which in use faces away from the skin surface, and a second side (5), which in use faces the skin surface, and where the silicone gel adhesive (4) has a layer thickness of at least 1500 µm to at most 3500 µm; and
b) a first film (2), which is joined indetachably to the first side (3) of the silicone gel adhesive (4) and which is non-adhesive on its side (1) facing away from the silicone gel adhesive;
where the sealing element has an adhesion strength of at least 8 N/25 mm and at most 20 N/25 mm, preferably of at least 10 N/25 mm and at most 20 N/25 mm, more preferably of at least 12 N/25 mm and at most 20 N/25 mm or, very preferably, of at least 16 N/25 mm and at most 20 N/25 mm, measured in analogy to DIN EN 1939 in conjunction with DIN ISO 6133:2004-05.

2. Apparatus for airtightly covering a wound for a negative-pressure therapy, comprising
a) an air-impermeable covering material (10) which can be affixed adhesively to a skin surface (7) bordering the wound, in such a way that the wound can be covered;
b) a sealing element according to Claim 1, which in use may be present between the covering material (10) and the skin surface (7) bordering the wound.

3. Sealing element or apparatus according to Claim 1 or 2, where the air-impermeable covering material (10) is a foil comprising a water-insoluble polymer, more particularly polyurethane, or a metal.

4. Sealing element or apparatus according to any of the preceding claims, where the strips in plan view are rectangular with dimensions of 5 cm × 10 cm to 10 cm × 18 cm.

5. Sealing element or apparatus according to any of the preceding claims, where the silicone gel adhesive (4) comprises a silicone gel based on polydimethylsiloxane.

6. Sealing element or apparatus according to any of the preceding claims, where the first film (2) comprises a polymer, more particularly polyurethane.

7. Sealing element or apparatus according to any of the preceding claims, where the first film (2) has a layer thickness of at least 20 µm to at most 60 µm.

8. Sealing element or apparatus according to any of the preceding claims, where the sealing element is provided with a second film (6) which is applied detachably on the second side (5) of the silicone gel adhesive (4).

9. Sealing element or apparatus according to Claim 8, where the second film (6) comprises a polymer, more particularly polyethylene.

10. Sealing element or apparatus according to Claim 8 or 9, where the second film (6) has a layer thickness of at least 100 µm to at most 120 µm.

11. Sealing element or apparatus according to any of the preceding claims, where the sealing element is provided in such a way that no further film for stabilization lies on the first film (2).

12. Sealing element or apparatus according to any of the preceding claims, where the sealing element is packaged in a pouch and provided in sterile form.

13. Apparatus according to any of Claims 2 to 12, where the apparatus further comprises
a) a wound dressing (13) which can be applied to the surface of the wound (12), so that the wound dressing in use is disposed between the surface of the wound (12) and the covering material (10),
b) a negative-pressure source (16), and
c) optionally means (14, 15) for functionally connecting the space (11) which is formed between the surface of the wound (12) and the covering material (10) to the negative-pressure source (16), so that a negative pressure can be produced in the space (11) between the surface of the wound (12) and the covering material (10), so that fluids secreted from the surface of the wound (12) can be removed from the space (11) between the surface of the wound (12) and the covering material (10).

## Revendications

1. Élément d'étanchéité, qui est préparé sous la forme de bandes, destiné à une utilisation dans un traitement par pression négative d'une plaie, comprenant un composite constitué par :
a) un adhésif à base de gel de silicone (4) pour la fermeture de fuites entre une surface de peau (7) adjacente à la plaie et un matériau de recouvrement imperméable à l'air (10), l'adhésif à base de gel de silicone (4) comprenant un premier côté (3) opposé à la surface de peau à l'usage et un deuxième côté (5) orienté vers la surface de peau à l'usage, et l'adhésif à base de gel de silicone (4) présentant une épaisseur de couche d'au moins 1 500 µm à au plus 3 500 µm ; et
b) un premier film (2) qui est relié de manière non amovible avec le premier côté (3) de l'adhésif à base de gel de silicone (4) et qui est configuré sous forme non adhésive sur son côté (1) opposé à l'adhésif à base de gel de silicone ;
l'élément d'étanchéité présentant une force d'adhésion d'au moins 8 N/25 mm et d'au plus 20 N/25 mm, de préférence d'au moins 10 N/25 mm et d'au plus 20 N/25 mm, de manière davantage préférée d'au moins 12 N/25 mm et d'au plus 20 N/25 mm ou de manière tout particulièrement préférée d'au moins 16 N/25 mm et d'au plus 20 N/25 mm, mesurée de manière analogue à DIN EN 1939 conjointement avec DIN ISO 6133:2004-05.

2. Dispositif pour le recouvrement étanche à l'air d'une plaie pour un traitement par pression négative, comprenant :
a) un matériau de recouvrement imperméable à l'air (10), qui peut être fixé par adhésion à une surface de peau (7) adjacente à la plaie de sorte que la plaie puisse être recouverte ;
b) un élément d'étanchéité selon la revendication 1, qui peut être présent à l'usage entre la surface de peau (7) adjacente à la plaie et le matériau de recouvrement (10) .

3. Élément d'étanchéité ou dispositif selon la revendication 1 ou 2, dans lequel le matériau de recouvrement imperméable à l'air (10) est un film comprenant un polymère insoluble dans l'eau, notamment un polyuréthane, ou un métal.

4. Élément d'étanchéité ou dispositif selon l'une quelconque des revendications précédentes, dans lequel les bandes sont configurées en vue du dessus sous forme rectangulaire avec les dimensions 5 cm x 10 cm à 10 cm x 18 cm.

5. Élément d'étanchéité ou dispositif selon l'une quelconque des revendications précédentes, dans lequel l'adhésif à base de gel de silicone (4) comprend un gel de silicone à base de polydiméthylsiloxane.

6. Élément d'étanchéité ou dispositif selon l'une quelconque des revendications précédentes, dans lequel le premier film (2) comprend un polymère, notamment un polyuréthane.

7. Élément d'étanchéité ou dispositif selon l'une quelconque des revendications précédentes, dans lequel le premier film (2) présente une épaisseur de couche d'au moins 20 µm à au plus 60 µm.

8. Élément d'étanchéité ou dispositif selon l'une quelconque des revendications précédentes, dans lequel l'élément d'étanchéité est préparé avec un deuxième film (6) qui est appliqué sous forme amovible sur le deuxième côté (5) de l'adhésif à base de gel de silicone (4).

9. Élément d'étanchéité ou dispositif selon la revendication 8, dans lequel le deuxième film (6) comprend un polymère, notamment un polyéthylène.

10. Élément d'étanchéité ou dispositif selon la revendication 8 ou 9, dans lequel le deuxième film (6) présente une épaisseur de couche d'au moins 100 µm à au plus 120 µm.

11. Élément d'étanchéité ou dispositif selon l'une quelconque des revendications précédentes, dans lequel l'élément d'étanchéité est préparé de sorte qu'aucun film supplémentaire pour la stabilisation n'est disposé sur le premier film (2).

12. Élément d'étanchéité ou dispositif selon l'une quelconque des revendications précédentes, dans lequel l'élément d'étanchéité est emballé dans un sac et préparé sous forme stérile.

13. Dispositif selon l'une quelconque des revendications 2 à 12, dans lequel le dispositif comprend en outre :
a) un pansement (13), qui peut être appliqué sur la surface de la plaie (12) de sorte que le pansement soit agencé à l'usage entre la surface de la plaie (12) et le matériau de recouvrement (10),
b) une source de pression négative (16) et
c) éventuellement des moyens (14, 15) pour le raccordement fonctionnel de l'espace (11) formé entre la surface de la plaie (12) et le matériau de recouvrement (10) avec la source de pression négative (16), de sorte qu'une pression négative puisse être générée dans l'espace (11) entre la surface de la plaie (12) et le matériau de recouvrement (10), de manière à ce que des liquides excrétés puissent être éliminés de la surface de la plaie (12) à partir de l'espace (11) entre la surface de la plaie (12) et le matériau de recouvrement (10) .
